# FASCICULE DE BREVET EUROPEEN

(11) **EP 1 649 011 B1**
(45) Date de publication et mention de la délivrance du brevet: **27.10.2010**
(21) Numéro de dépôt: 04767688.7
(22) Date de dépôt: 15.07.2004
(51) Int. Cl.: C12N 5/071, C12N 7/02, A61K 39/255

(54) **PROCEDE DE CULTURE DE KERATINOCYTES ET SES APPLICATIONS**
KERATINOZYTEN-KULTURVERFAHREN UND VERWENDUNG DAVON
CULTURE METHOD FOR KERATINOCYTES AND USE THEREOF

(30) Priorité: 18.07.2003 FR 0308781
(43) Date de publication de la demande: 26.04.2006
(73) Titulaire: CENTRE NATIONAL DE LA RECHERCHE SCIENTIFIQUE (CNRS), 75016 Paris (FR)
(72) Inventeur: DJIAN, Philippe, F-75015 Paris (FR); VANHOUTTEGHEM, Amandine, Laetitia, Vanessa, F-75009 Paris (FR)
(74) Mandataire: Vialle-Presles, Marie José
(86) Numéro de dépôt international: PCT/FR2004/001864
(87) Numéro de publication internationale: WO 2005/010173

(56) Documents cités:
- EP-A- 0 496 135
- EP-A- 0 703 294
- EP-A- 0 748 867
- EP-A- 0 770 677
- EP-A- 0 779 359
- EP-A- 1 064 947
- EP-A- 1 149 899
- US-A- 6 001 369
- US-A- 6 001 369
- BEELE H ET AL: "In-vitro culture of chick down feather bulbi: A tool to obtain proliferating and differentiating keratinocytes in an organotypic structure" EXPERIENTIA, vol. 46, no. 10, 1990, pages 1053-1057, XP002312709 ISSN: 0014-4754
- MEYERS C ET AL: "Synthesis of infectious human papillomavirus type 18 in differentiating epithelium transfected with viral DNA." JOURNAL OF VIROLOGY, vol. 71, no. 10, octobre 1997 (1997-10), pages 7381-7386, XP002272429 ISSN: 0022-538X cité dans la demande
- ABUJOUB A ET AL: "Development of a sustainable chick cell line infected with Marek's disease virus." VIROLOGY, vol. 214, no. 2, 20 décembre 1995 (1995-12-20), pages 541-549, XP000652237 ISSN: 0042-6822 cité dans la demande
- VANHOUTTEGHEM A ET AL: "Serial cultivation of chicken keratinocytes, a composite cell type that accumulates lipids and synthesizes a novel beta-keratin" DIFFERENTIATION, vol. 72, no. 4, avril 2004 (2004-04), pages 123-137, XP002312710 ISSN: 0301-4681
- BOULANGER D ET AL.: "Morphogenesis and release of fowlpox virus" JOURNAL OF GENERAL VIROLOGY, vol. 81, no. Pt.3, March 2000 (2000-03), pages 675-687,
- SAINI S S AND AL.: "Immune response of chicks to oral vaccination with combined extra- and intracellular fowl pox viruses" TROPICAL ANIMAL HEALTH & PRODUCTION, vol. 22, August 1990 (1990-08), pages 165-169,
- LAIDLAW S M ET AL.: "Fowlpox virus encodes nonessential homologs of cellular alpha-SNAP, PC-1, and an orphan human homolog of a secreted nematode protein." JOURNAL OF VIROLOGY, vol. 72, no. 8, August 1998 (1998-08), pages 6742-6751,
- BOULANGER D. ET AL: 'Morphogenesis and release of fowlpox virus' JOURNAL OF GENERAL VIROLOGY vol. 81, no. PT.3, Mars 2000, pages 675 - 687
- SAINI S.S. ET AL: 'Immune response of chicks to oral vaccination with combined extra- and intracellular fowl pox viruses' TROPICAL ANIMAL HEALTH & PRODUCTION vol. 22, Août 1990, pages 165 - 169
- LAIDLAW S.M. ET AL: 'Fowlpox virus encodes nonessential homologs of cellular alpha-SNAP, PC-1, and an orphan human homolog of a secreted nematode protein.' JOURNAL OF VIROLOGY vol. 72, no. 8, Août 1998, pages 6742 - 6751

### SEQUENCE LISTING

<110> CENTRE NATIONAL DE LA RECHERCHE SCIENTIFIQUE
<120> NOUVEAU PROCEDE DE CULTURE DE KERATINOCYTES ET SES APPLICATIONS
<130> VCstsF644/110FR
<160> 8
<170> PatentIn version 3.1
<210> 1
   <211> 20
   <212> PRT
   <213> Artificial sequence
<220>
   <223> Nucleotide
<400> 1
   Ala Gly Thr Gly Gly Ala Thr Thr Cys Thr Ala Thr Gly Gly Ala Cys
   1               5                   10                  15
   Cys Thr Gly Cys
               20
<210> 2
   <211> 19
   <212> PRT
   <213> Artificial sequence
<220>
   <223> Nucleotide
<400> 2
   Ala Ala Cys Thr Gly Ala Gly Cys Ala Gly Cys Thr Thr Gly Cys Thr
   1               5                   10                  15
   Gly Gly Cys
<210> 3
   <211> 19
   <212> PRT
   <213> Artificial sequence
<220>
   <223> Nucleotide
<400> 3
   Cys Ala Gly Thr Cys Cys Ala Gly Gly Thr Ala Cys Gly Ala Ala Gly
   1 5 10 15
   Ala Gly Cys
<210> 4
   <211> 20
   <212> PRT
   <213> Artificial sequence
<220>
   <223> Nucleotide
<400> 4
   Thr Ala Gly Gly Ala Thr Cys Gly Gly Gly Ala Thr Ala Thr Gly Gly
   1 5 10 15
   Ala Ala Gly Cys
   20
<210> 5
   <211> 20
   <212> PRT
   <213> Artificial sequence
<220>
   <223> Nucleotide
   <400> 5
   Gly Gly Cys Thr Thr Thr Gly Ala Thr Gly Cys Thr Ala Thr Cys Thr
   1 5 10 15
   Gly Thr Gly Cys
   20
<210> 6
   <211> 19
   <212> PRT
   <213> Artificial sequence
<220>
   <223> Nucleotide
<400> 6
   Gly Thr Gly Gly Ala Gly Thr Cys Thr Gly Ala Cys Ala Thr Cys Ala
   1               5                   10                  15
   Ala Cys Gly
<210> 7
   <211> 20
   <212> PRT
   <213> Artificial sequence
<220>
   <223> Nucleotide
<400> 7
   Ala Gly Ala Ala Cys Thr Gly Ala Gly Ala Cys Gly Cys Ala Cys Gly
   1               5                   10                  15
   Ala Thr Gly Cys
               20
<210> 8
   <211> 20
   <212> PRT
   <213> Artificial sequence
<220>
   <223> Nucleotide
<400> 8
   Ala Cys Thr Ala Gly Gly Ala Thr Gly Thr Thr Ala Cys Thr Gly Cys
   1               5                   10                  15
   Gly Thr Gly Gly
               20

## Description

La présente invention a pour objet un nouveau milieu de culture de kératinocytes, un procédé de culture de kératinocytes et l'utilisation de ce procédé pour la propagation de virus vivants atténués. Les virus vivants atténués obtenus par cette méthode et les vaccins les comprenant sont décrits.

Un certain nombre de virus pathogènes ne peuvent se répliquer de façon complète que dans les kératinocytes, car des protéines kératinocytaires spécifiques sont indispensables à leur réplication.

C'est le cas par exemple du MDV (Marek's disease virus ou virus herpès responsable de la maladie de Mareck aviaire) et du FPV (Fowl pox virus ou virus pox responsable de la variole aviaire).

Les cellules utilisées actuellement pour la propagation du virus du MDV sont en général des fibroblastes. Les fibroblastes de poulet en culture utilisés pour la production de vaccins contre le MDV n'assurent pas la production de particules virales libres : l'infection est semi-productive. Des tentatives ont été menées pour tenter d'établir des lignées continues capables d'infection productive, mais sans succès (Abujoub et al., Virology, 214, 541-549, 1995). En raison du caractère incomplet de la réplication virale dans les fibroblastes, les vaccins cellulaires doivent impérativement être utilisés sous forme congélée dans l'azote liquide. Comme le virus produit par les fibroblastes est dépourvu d'une enveloppe complète, il n'est ni cytolytique, ni infectieux.

Dans le cas de la maladie de Marek, l'infection de l'animal se fait par voie respiratoire. Peu après la contamination, une infection cytolytique des lymphocytes B se développe, dont la conséquence est une virémie cellulaire qui aboutit à l'infection latente de nombreux tissus. Parmi les types cellulaires infectés, on trouve les lymphocytes T qui vont ensuite se transformer et produire des lymphomes mortels. Le virus est également transporté vers l'épiderme au niveau des follicules des plumes qui représentent le seul site de production de virus ayant une enveloppe complète. Le virus est présent à des titres très élevés dans les cellules comifiées (squames) de la partie superficielle de l'épiderme, en particulier au niveau des follicules des plumes. Ces cellules desquament et infectent les autres animaux par inhalation des squames (Calnek et al., J. Natl. Cancer Inst., 45, 341-351, 1970 ; Calnek et al., Avian Dis., 14, 219-233, 1970 ; Carrozza et al., Avian Dis. 17, 767-781, 1973). De nombreux vaccins ont été développés, car la maladie de Marek est une maladie présente dans le monde entier, responsable de lourdes pertes dans les élevages atteints. Ces vaccins sont constitués d'une suspension de fibroblastes de poulets infectés en culture par des souches atténuées de virus de la maladie de Marek. Les vaccins sont préparés par congélation dans l'azote liquide des cellules vivantes dans du milieu de culture contenant des cryoprotecteurs. La vaccination se fait soit par injection *in ovo*, soit par injection sous-cutanée, un jour après la naissance. La vaccination protège contre le syndrome lympho-prolifératif, mais pas contre l'infection des kératinocytes. Ceci explique la persistance et la transmission continues du virus. Les mutations rapides du virus dues à sa persistance entraînent l'apparition de formes de plus en plus virulentes résistantes aux vaccins existants.

Les virus humains du papillome sont de petits virus à ADN dont le cycle biologique est associé à la différentiation terminale des kératinocytes des épithélium stratifiés squameux. Les gènes régulateurs et non structuraux sont exprimés dans la couche basale des épithélium stratifiés, mais la réplication de l'ADN, la synthèse des protéines de structure et l'assemblage de la capside se font dans les couches superficielles, autrement dit dans les kératinocytes engagés dans leur processus de différentiation terminale. Les sous types HPV 16 et 18 sont retrouvés dans plus de 90% des cancers du col de l'utérus et l'on admet qu'ils soient impliqués dans les causes de ce cancer (zur Hausen, H., J. Natl. Cancer Inst., 92, 690-698, 2000). Le cancer du col de l'utérus représente un problème de santé publique majeur, ce qui a conduit à d'importants travaux visant au développement de vaccins prophylactiques (Plummer et al., Virus Res., 89, 285-293, 2002). Toutefois, le développement de ces vaccins a été ralenti par l'absence de méthode de culture cellulaire assurant la réplication efficace et la production de virions.

Il subsiste donc le besoin d'un procédé permettant d'assurer la réplication complète de certains virus pathogènes, notamment les virus à ADN enveloppés, tels que par exemple le MDV et le FPV.

Sachant que des protéines kératinocytaires spécifiques sont indispensables à la réplication virale, des tentatives ont été entreprises pour cultiver des kératinocytes dans le but d'assurer la production de virus atténués complets :
Si les kératinocytes humains peuvent être cultivés (Rheinwald et al., Cell, 6, 331-343, 1975), leur état de différentiation en culture est incomplet. Meyers et al., J.Virol., 71, 7381-7386, 1997 décrit la synthèse de virions HPV infectieux. Des kératinocytes sont cultivés sur des cellules 3T3 traitées à la mitomycine dans un milieu de culture nommé milieu E qui est un milieu synthétique dépourvu de sérum de poulet.

Toutefois, le HPV est distinct du MPV et la méthode de synthèse de virions HPV décrite dans ce document ne permet pas de produire des virions de la maladie de Marek.

Les documents EP 1 064 947, EP 0 703 294 et EP 0 496 135 décrivent des virions acellulaires atténués du MDV.

Le document D. BOULANGER et al, Journal of General Virology, 81, 2000, 675-687, décrit un virus acellulaire de la souche FP9 du FPV.

Le document EP-0 748 867 décrit des cultures du virus de la maladie de Marek de sérotype 1 sur une lignée cellulaire continue, la lignée QT35. Les cellules infectées sont utilisées pour la préparation d'un vaccin et non pas d'un virion isolé, car ce virus est fortement associé à la cellule. Produit avec une enveloppe incomplète il ne peut pas être isolé tout en restant infectieux.

Le document EP-0 770 677 décrit la propagation du virus de la maladie de Marek du sérotype 2 sur des lignées cellulaires de poulet. Les virions obtenus sont dépendants de la culture cellulaire.

Le document EP-1 149 899 décrit un procédé de culture de cellules ES d'oiseau. Le milieu de culture contient préférentiellement un tapis de cellules nourricières, telles que des cellules STO qui sont des fibroblastes d'embryon de souris, inactivées par traitement à la mitomycine ou par irradiation. Il contient aussi du sérum de poulet.

Le document EP-0 779 359 décrit un procédé de culture de cellules aviaires. Le milieu de culture contient du sérum de poulet. La culture est supportée par des cellules STO (fibroblastes) ou SL-10.

Il apparaît donc que toutes les cultures de kératinocytes ne permettent pas d'offrir des conditions permettant d'assurer une réplication virale complète, notamment les virus à ADN enveloppés tels que le MDV.

D'autre part, si pratiquement tous les types cellulaires humains et animaux peuvent être cultivés avec succès en présence de sérum bovin (on peut par exemple se reporter à Dorange et al., J.Virol., 76, 1959-1970, 2002), les kératinocytes de poulet ne se multiplient pas dans ces conditions.

L'objectif de la présente invention était de mettre au point des conditions permettant de cultiver des kératinocytes, notamment des kératinocytes aviares et plus particulièrement des kératinocytes de poulet, de telle sorte que ces cellules conservent l'ensemble des propriétés de différentiation qu'elles possèdent *in vivo*.

Le cycle de réplication des virus qui infectent des cellules hautement différenciées (hépatocytes, entérocytes, cellules neuronales ou kératinocytes) étant généralement lié au programme de différenciation de sa cellule hôte (Calnek et al., J. Natl. Cancer Inst., 45, 341-351, 1970 ; Calnek et al., Avian Dis., 14, 219-233, 1970 ; Schmitt et al., J. Virol., 70, 1912-1922, 1996), on peut attendre de cultures de kératinocytes totalement différenciées qu'elles permettent de produire une réplication virale complète et donc des virions dépourvus de cellules.

L'invention décrit un milieu de culture de cellules épidermiques, notamment de cellules épidermiques de volailles, comprenant :
(i) du sérum de poulet,
   et
(ii) un support de culture comprenant des fibroblastes inactivées par un traitement antimitotique.

L'invention a pour objet une culture de kératinocytes comportant un milieu de culture qui comprend du sérum de poulet et un support de culture comprenant des cellules de mammifères inactivées, préférentiellement des fibroblastes inactivés.

En outre, le milieu de culture de l'invention comprend de façon connue un ou plusieurs additifs qui peuvent être sélectionnés parmi les sels inorganiques, les vitamines, les hormones et les facteurs de croissance ou tout autre additif habituellement utilisé dans les milieux de culture de cellules. Parmi les additifs utilisables dans le milieu de culture de l'invention, on peut citer en particulier : les sels inorganiques, les acides aminés, les vitamines, les acides gras, le glucose, un tampon, du rouge de phénol, de l'EGF (epidermal growth factor ou facteur de croissance épidermique), du T₃, de l'hydrocortisone, de l'insuline, de la toxine cholérique, de la transferrine, de l'adénine...

Les fibroblastes utilisées comme support de culture sont avantageusement des cellules 3T3, et de façon encore plus avantageuse le sous clone 3T3-J2. L'origine de la lignée 3T3 est décrite dans Todaro, G., J. et al., J.Cell Biol., 17, 299-313, 1963. La lignée cellulaire 3T3 est enregistrée dans la collection ATCC sous le numéro CCL92. Les cellules 3T3 sont avantageusement utilisées dans l'invention en une monocouche de cellules qui sont inactivées par un traitement antimitotique. Tout traitement connu pour son effet antimitotique peut être utilisé. Ce traitement peut consister en un traitement par un antibiotique tel que la mitomycine C ou une irradiation létale aux rayons gamma.

Dans le milieu de culture de l'invention, le sérum de poulet est avantageusement présent en une proportion en poids par rapport au poids total du milieu de culture allant de 0,5 à 50%, et préférentiellement de 1 à 40%, avantageusement de 2 à 30%.

Avantageusement, le milieu de croissance pour la culture cellulaire, contient au moins un et de façon avantageuse plusieurs des additifs suivants : de la toxine cholérique, de la transferrine, de l'adénine, de 1' EGF, des hormones telles que T3, de l'hydrocortisone, de l'insuline bovine.

La présente invention se rapporte également à un procédé de culture de kératinocytes, et plus particulièrement de kératinocytes aviaires, ledit procédé comprenant les étapes consistant à :
(i) préparer une suspension de cellules épidermiques ou kératinocytes ;
(ii) inoculer lesdites cellules sur un support cellulaire constitué de cellules 3T3 dans un milieu de culture comprenant du sérum de poulet tel que décrit ci-dessus ;
(iii) incuber lesdites cellules épidermiques sur leur support cellulaire.

La suspension de cellules épidermiques, ou kératinocytes, peut être obtenue de façon connue par digestion enzymatique d'un épiderme. Une telle digestion peut être effectuée par traitement par une enzyme protéolytique comme par exemple par traitement par la trypsine, par la dispase, par la thermolysine. On peut également envisager de traiter l'épiderme par un traitement chimique comme par exemple un traitement à l'EDTA (éthylène diamine tétraacétate).

Les conditions d'incubation sont celles habituellement appliquées à une culture de cellules, c'est-à-dire une température d'environ 37°C sous 5 à 10% de CO2 et 100% d'humidité.

Après incubation, lorsque la densité cellulaire est suffisante, les cellules obtenues dans ces conditions peuvent être transférées sur un nouveau support cellulaire et à nouveau mises en culture dans les conditions décrites ci-dessus. Lorsqu'à l'issue de l'étape (iii) les cellules issues de la culture sont récupérées, on leur applique un nouveau cycle de culture suivant les étapes (ii) et (iii) du procédé de l'invention. On peut prévoir jusqu'à 30 cycles de multiplication cellulaire successifs suivant le procédé décrit ci-dessus. Le temps de doublement de la population cellulaire est d'environ 12 à 16 heures lors des dix premiers cycles de multiplication cellulaire, puis il augmente progressivement lors des cycles suivants. Par exemple, en partant de 10⁶ cellules inoculées au départ, on peut obtenir environ 10¹² cellules, soit un facteur d'expansion d'environ 10⁶.

Les cellules obtenues après 1 ou plusieurs cycles de culture peuvent également être conservées sous forme congelée en vue de leur utilisation ultérieure. Les cultures cellulaires comprenant un support de cellules 3T3, un milieu de culture comprenant du sérum de poulet et des kératinocytes constituent un autre objet de l'invention.

De façon surprenante, il a pu être démontré que le procédé de culture de l'invention permet de conserver aux kératinocytes issus de cette culture toutes leurs caractéristiques de différentiation :
L'épiderme aviaire est un épithélium stratifié squameux riche en lipides et en filaments d'alpha et de béta kératine. Les cellules de la couche inférieure reposent sur une membrane basale formée de protéines dont certaines, comme la laminine 5, sont des produits spécifiques des kératinocytes. La couche basale contient les cellules souches kératinocytaires qui synthétisent des marqueurs spécifiques: p63. La cohésion de l'épiderme est assurée par des liaisons inter-kératinocytaires comme les desmosomes et les jonctions communicantes contenant respectivement les protéines plakoglobine et connexine 31. Dans les couches superficielles de l'épiderme, les kératinocytes subissent un processus de différenciation terminale au cours de laquelle elles synthétisent 14-3-3 sigma et forment une enveloppe cornée qui reste insoluble après ébullition dans les détergents et les agents réducteurs. Cette enveloppe contient des protéines spécifiques comme la périplakine.

Par le procédé de culture de l'invention les kératinocytes de poulet en culture synthétisent des lipides dont la composition est voisine de celle de l'épiderme aviaire, des filaments d'alpha et de béta kératine, des desmosomes, les protéines p63, 14-3-3 sigma, plakoglobine, connexine 31 et périplakine. Enfin les kératinocytes de poulet forment en culture des colonies stratifiées dans les couches superficielles desquelles les cellules synthétisent une enveloppe cornée insoluble dans les détergents et les agents réducteurs. Par conséquent, dans le système de culture selon l'invention, les kératinocytes maintiennent l'ensemble des propriétés qu'ils possèdent *in vivo*.

La possibilité d'obtenir des cultures de kératinocytes dont les propriétés de différentiation sont conservées permet d'envisager de propager des virus dans des conditions de réplication virale complètes. Dans le cas du MDV par exemple, les kératinocytes de poulet constituent le seul type cellulaire qui peut assurer une réplication complète du virus et la production de virions entourés d'une enveloppe entière. Du fait des propriétés cytolytiques de ce virus, celui-ci est libéré à l'extérieur de la cellule infectée (Biggs, P.M., Marek's disease, ed. Springer-Verlag, 1-24, 2001) et il peut donc être facilement extrait du milieu de culture. De plus, ces cellules produisent des quantités de virus très importantes, ce qui facilite la production à grande échelle de ces virus.

Par conséquent, l'invention a encore pour objet un procédé de propagation d'un virus *in vitro,* ce procédé étant **caractérisé en ce qu**'il comprend les étapes consistant à :
(i) infecter des kératinocytes avec la souche du virus ;
(ii) mettre en culture les kératinocytes infectés selon le procédé de culture précédemment décrit ;
(iii) extraire les virions produits à l'étape (ii).

Le procédé de l'invention permet la préparation de particules virales acellulaires qui peuvent être lyophilisées après extraction et donc permettre leur conservation et leur stockage dans des conditions plus favorables que les suspensions de cellules infectées de l'art antérieur. Le virus atténué par plusieurs cycles de culture est néanmoins produit sous forme de virions complets. Cette caractéristique permet d'envisager de préparer des vaccins plus efficaces, apportant une meilleure immunisation que ceux de l'art antérieur. De préférence on met en oeuvre le procédé de propagation de virus en effectuant de 10 à 400 cycles de culture, avantageusement de 40 à 100 cycles de culture. Les virions à ADN enveloppés vivants atténués issus du procédé décrit ci-dessus sont décrits.

L'invention a encore pour objet un procédé de préparation d'un vaccin, ce procédé étant **caractérisé en ce qu'**il comporte les étapes consistant à propager le virus suivant la méthode décrite ci-dessus, puis à introduire le virion dans un support pharmaceutiquement acceptable, en particulier un support de vaccin. On prévoit avantageusement que le virion soit lyophilisé et stocké avant d'être introduit dans le support pharmaceutiquement acceptable. Notamment le vaccin peut être transporté à température ambiante. Le support de vaccin peut contenir de façon connue les adjuvants de vaccins habituellement employés.

Le procédé de l'invention s'applique plus particulièrement à la production de vaccins à partir de virus à ADN enveloppé , on peut citer en particulier les virus MDV, FPV.

La présente invention décrit également un vaccin atténué vivant comprenant un virion obtenu à partir d'un virus à ADN enveloppé. De façon classique, un tel vaccin comprend un support de vaccin sous forme d'une solution aqueuse comprenant de préférence un ou plusieurs adjuvants de vaccin. Un tel vaccin est généralement commercialisé sous forme d'un kit de vaccination, comprenant d'une part le virion lyophilisé, d'autre part le support de vaccination, chacun étant conditionné dans un compartiment séparé, les deux composants étant mélangés de façon extemporanée en vue de leur administration par voie orale ou par injection, ou par voie *in ovo.*

On peut également prévoir que les vaccins de l'invention soient administrables par voie respiratoire, dans ce cas ils sont préparés sous forme de solution aqueuse ou d'aérosol.

La possibilité de cultiver des kératinocytes permet de développer des souches de virus atténuées capables de réplication dans les kératinocytes. Dans le cas du virus MDV, responsable de la maladie de Marek, après administration de ce vaccin à des volailles d'un élevage, ces virions seraient présents dans les squames et donc susceptibles d'infecter et de protéger l'ensemble d'un élevage. L'invention a en outre pour objet l'utilisation d'un virion atténué pour la préparation d'un médicament destiné à la prévention ou au traitement de la maladie de Marek.

Le procédé de culture de kératinocytes de l'invention trouve d'autres applications dans le domaine de la recherche :
La culture de kératinocytes doit permettre d'identifier le mécanisme de pénétration d'un virus dans ce type cellulaire et en particulier d'identifier les protéines de l'enveloppe virale nécessaires à cette pénétration. Ces protéines pourront ensuite être utilisées sous forme recombinante pour l'immunisation des animaux. Dans le cas de la maladie de Marek, la séquence nucléotidique complète DU VIRUS responsable de cette maladie est connue. La sélection de virus mutants incapables d'infecter les kératinocytes permettra de trouver les mutations correspondantes et donc les protéines responsables de la pénétration dans les kératinocytes.

La présente invention va être davantage décrite et illustrée dans la suite par des exemples, qui sont fournis seulement à titre d'illustration de l'invention et ne devraient pas être considérés comme limitant celle-ci :

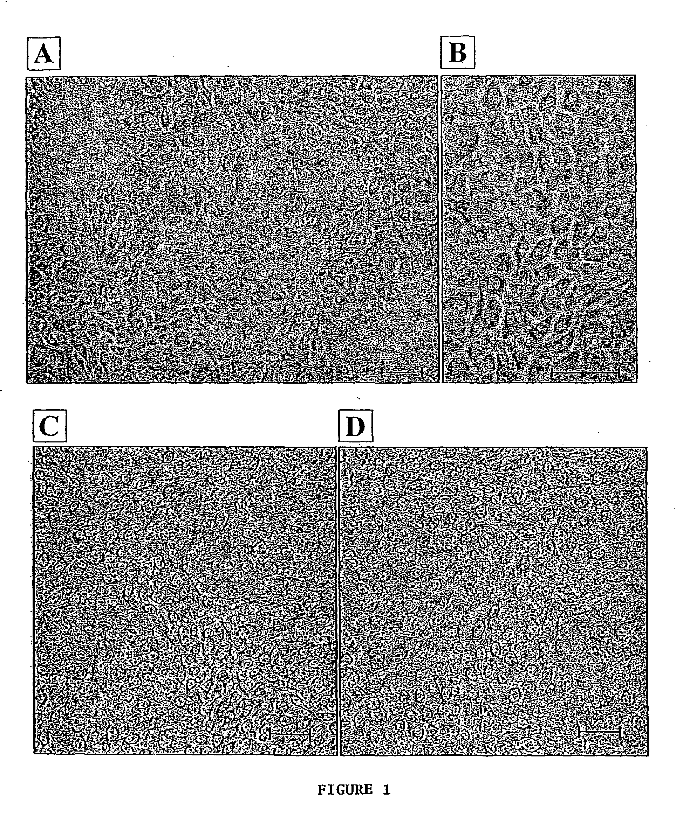

- la figure 1 représente des photographies en microscope de phase qui illustrent la croissance des cellules épidermiques de poulet et humaines : (A) 10⁵ cellules épidermiques de poulet lors de leur 3^{ème} passage sont mises en culture dans un milieu contenant 10% de sérum de poulet ; (B) la même culture que dans (A) prise avec un grossissement supérieur; (C) 7,8x10⁵ kératinocytes humains (YF23) sont mis en culture dans un milieu contenant 10% de sérum de veau foetal; (D) les kératinocytes humains (YF23) sont mis en culture dans un milieu contenant 10% de sérum de poulet ; chaque barre d'échelle correspond à 50 µm ;

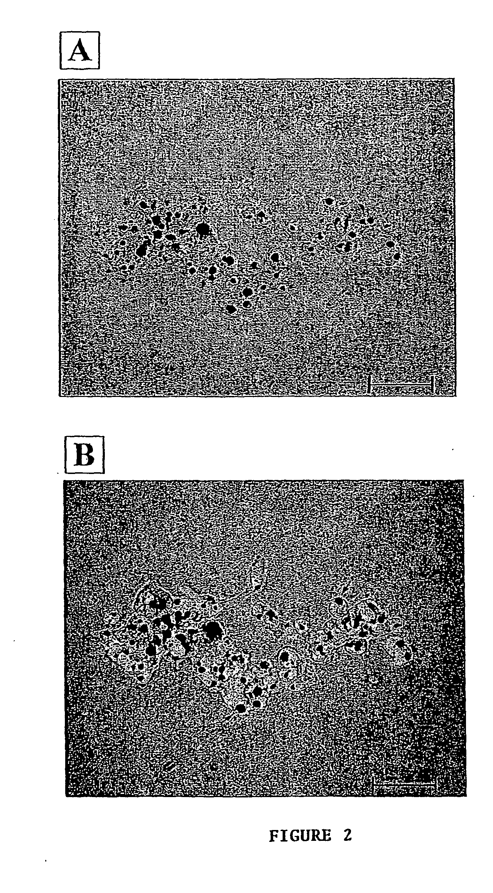

- la figure 2 illustre l'accumulation de lipides cytoplasmiques par les kératinocytes en culture après marquage par du Oil Red-O et coloration par le réactif de Hoechst 33258 ; (A) Microscopie en phase avec un filtre jaune ; (B) Photographie du même champ pris à la fois en contraste de phase et en fluorescence et imprimée en noir et blanc ; les flèches montrent les noyaux colorés par le réactif de Hoechst 33258 (blanc) ; l'échelle correspond à 25 µm ;

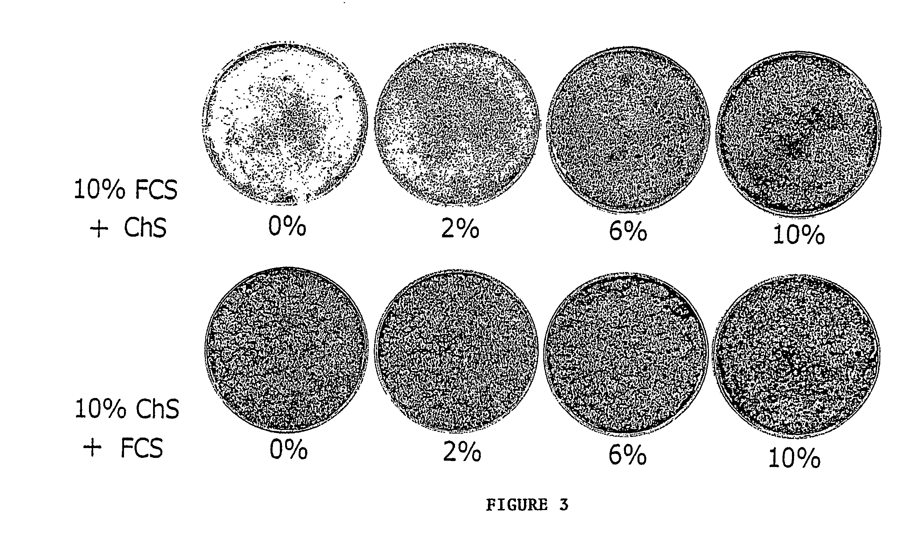

- la figure 3 illustre l'effet du sérum de poulet sur la multiplication des kératinocytes de poulet ; 5x10⁴ cellules sont mises en culture dans un mélange de sérum de poulet (ChS) et de sérum de veau foetal (FCS), puis elles sont fixées et colorées par la rhodamine B ; Dans un cas le pourcentage de FCS dans le milieu de culture est fixé à 10% et le pourcentage de ChS est variable. Dans l'autre cas, le pourcentage de ChS dans le milieu de culture est fixé à 10% et le pourcentage de FCS est variable.

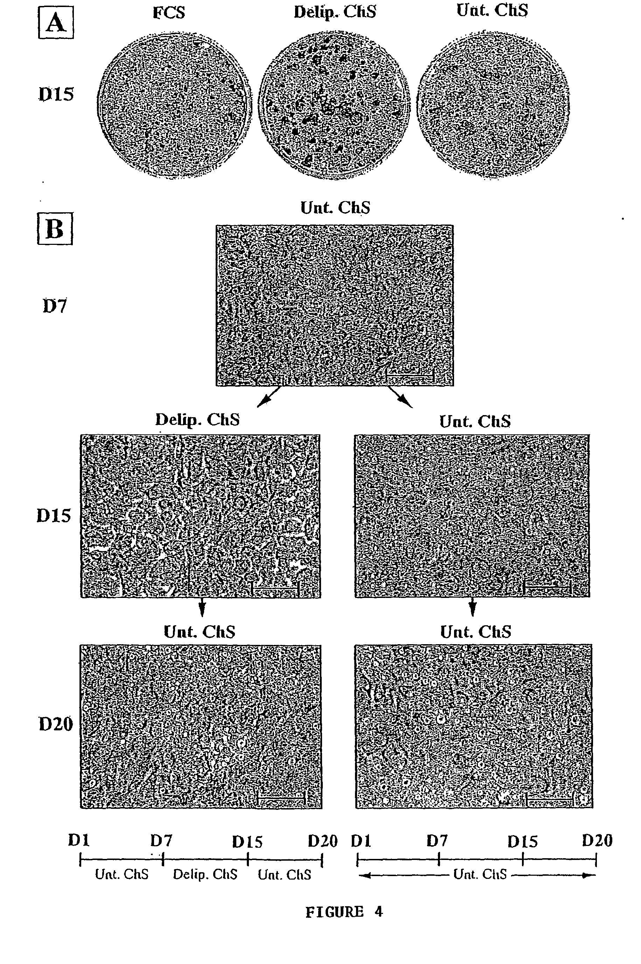

- la figure 4 illustre le fait que la délipidation du sérum de poulet n'affecte pas la multiplication des kératinocytes de poulet; des kératinocytes de poulet, avec une densité de 3,500 cellules/cm², sont inoculés et mis en culture dans 10% de sérum de poulet pendant 7 jours ; dans quelques boites, le supplément en sérum est remplacé, soit par du sérum de veau foetal (FCS), soit par du sérum de poulet délipidé (Delip.ChS), alors que les cellules des autres boites sont maintenues dans du sérum de poulet non-traité (Unt. ChS) ; D1 : 1^{er} jour, D7 : 7^{ième} jour, D 15 : 15^{ième} jour, D20 : 20^{ième} jour ; (A) les cultures sont fixées 15 jours après l'inoculation et colorées par la rhodamine B ; les flèches indiquent des petites gouttelettes observés dans du sérum délipidé ;

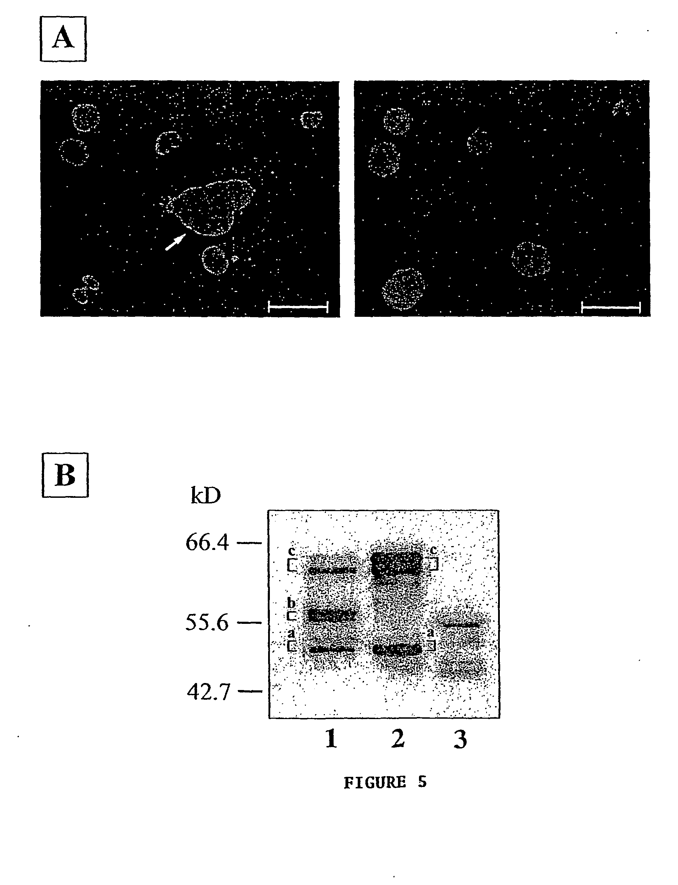

- la figure 5 illustre la mise en évidence des kératines dans les kératinocytes de poulet en culture ; (A) les cellules sont marquées par un anticorps anti-pankératine ; la flèche indique une cellule 3T3, qui est multi-nucléée et n'est pas marqué par l'anticorps ; l'échelle correspond à 25 µm ; (B) immunoblot marquée par un autre anticorps anti-pankératine ; la ligne (1) correspond aux kératinocytes de poulet en culture ; la ligne (2) correspond à l'épiderme de poulet et la ligne (3) correspond à l'intestin de poulet ; les deux doublets (a et c) coïncident avec les doublets correspondants dans l'épiderme de poulet ;

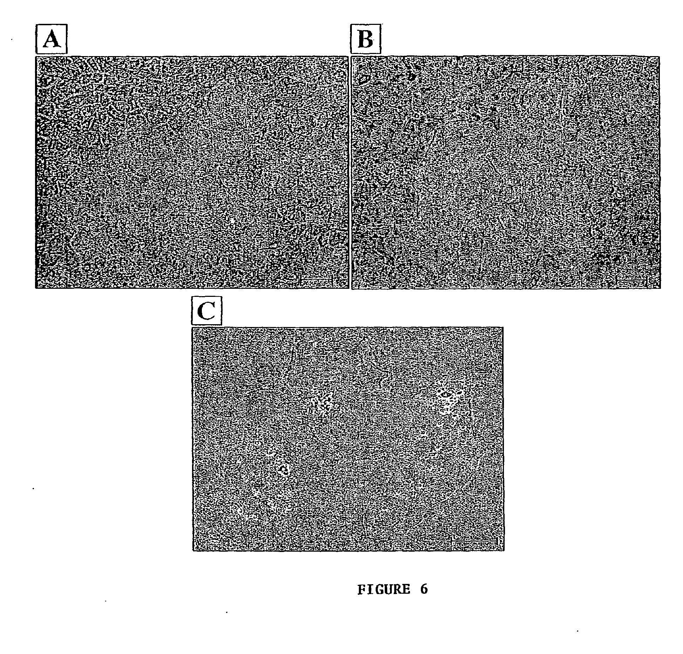

- la figure 6 montre que les kératinocytes de poulet forment des enveloppes comifiées en culture ; les photographies sont prises en microscopie en contraste de phase ; (A) couche basale d'une colonie vivante de 15 jours, (B) la même colonie prise sur un plan focal plus haut ; les contours sombres et la forme anguleuse indiquent une cornification, (C) après traitement à 100°C dans une solution contenant 2% de sulfate de dodecyl sodique et 2% de 2-mercaptoéthanol ; l'échelle correspond à 50 µm ;

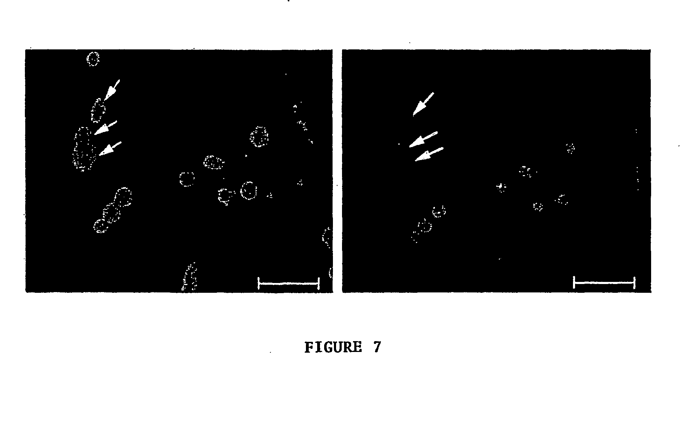

- la figure 7 illustre un marquage de la p63 par coloration immunofluorescence à l'aide d'anticorps anti-p63 ; les flèches indiquent les cellules 3T3 que l'on reconnaît à leurs gros noyaux et leur hétérochromatine proéminente ; elles ne sont pas marquées par l'anticorps ; l'échelle correspond à 25 µm ;

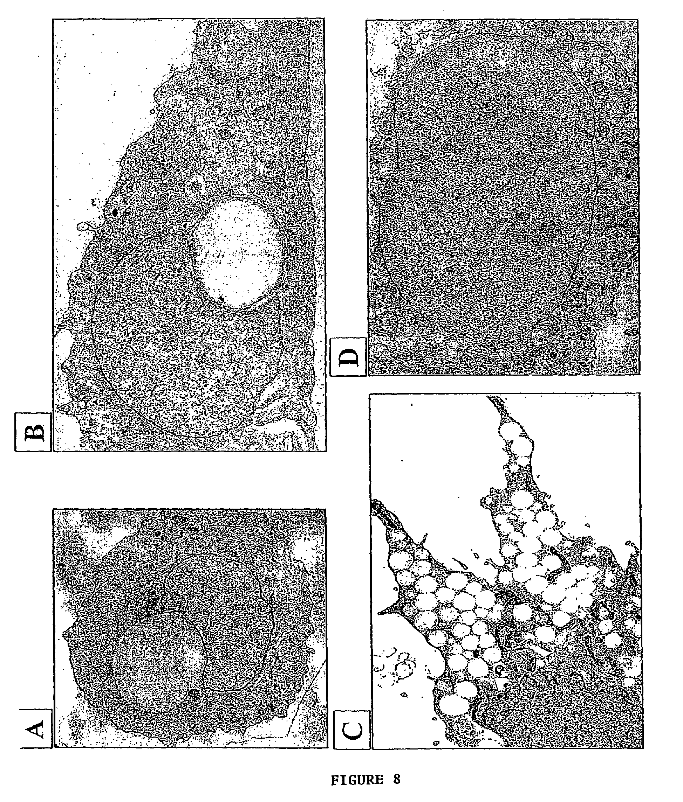

- la figure 8 montre des lipides péri- et intra-nucléaires ; ce sont des micrographies électroniques ; échelles de grossissement : 5000X pour A et B, 6000X pour C et 4000X pour D ;

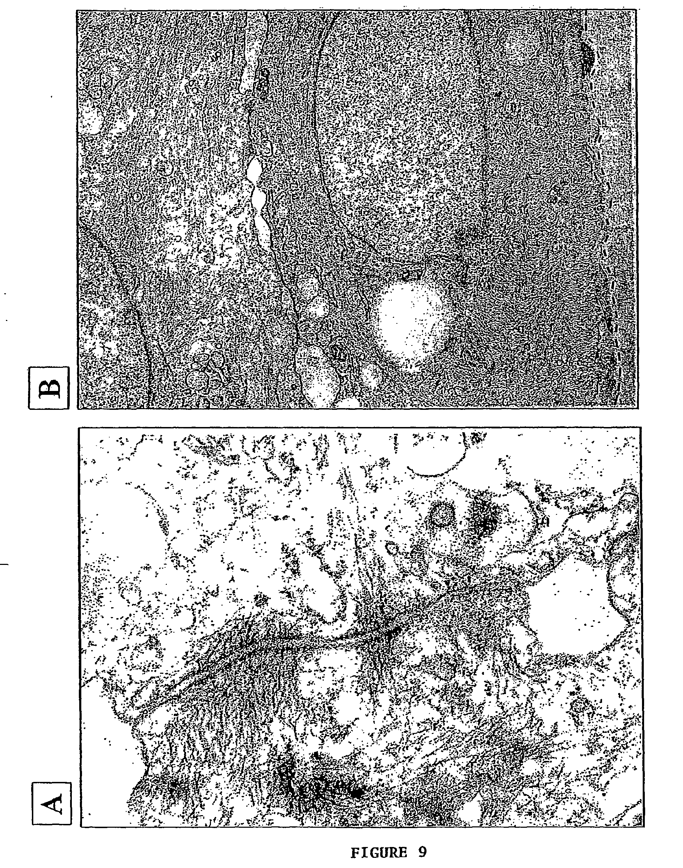

- la figure 9 montre la stratification dans les kératinocytes ; les desmosomes (en A) et les filaments de kératine (en B) ; ce sont des micrographies électroniques ; échelles de grossissement : 40000X pour A et 8000X pour B ;

### Example 1 : Multiplication des kératinocytes de poulet avec des cellules 3T3-J2 de support en présence de 10% de sérum de poulet

### 1.1 Introduction

La croissance des fibroblastes de poulet n'est pas supprimée de façon efficace par la monocouche de cellules 3T3 et les cultures préparés à partir de peau de poulet à épaisseur complète sont rapidement envahies par les fibroblastes. On a donc séparé l'épiderme du derme par traitement à la thermolysine avant de procéder à la désagrégation de l'épiderme. Initialement, le milieu avait été supplémenté avec du sérum de veau foetal, mais ce sérum ne permet pas la multiplication des kératinocytes de poulet. La substitution du sérum de bovin par du sérum de poulet permet le développement de colonies de cellules.

### 1.2 Matériel et Méthodes

### 1.2.1 Matériel biologique

Des poussins nouveaux-nés (*Gallus gallus*) sont obtenus d'oeufs fertilisés incubés pendant 20-21 jours à 37,8°C à taux humidité élevé. Pour préparer les cultures primaires de cellules épidermiques, les poussins nouveaux-nés sont anesthésiés par injection intra-péritonéale de tribromoéthanol.

Cellules murines 3T3-J2 : L'origine de la lignée 3T3 est décrite dans Todaro, G., J. et al., J.Cell Biol., 17, 299-313, 1963. Les cellules Swiss 3T3 d'origine obtenues de l'ATCC (n°CCL92) sont inoculées à faible densité dans des plaques de 96 puits (à raison d'une cellule pour trois puits). Après environ deux semaines de culture, certains puits contiennent des colonies cellulaires visibles. Ces cultures sont clonales car dérivées d'une seule cellule, chacune constitue donc un sous clone de 3T3. Chaque colonie est transférée séparément dans une boîte de culture de 100mm de diamètre. Lorsque le nombre de cellules est suffisant, les cellules sont détachées par trypsination. La moitié de chaque sous clone est congelée dans l'azote liquide ; l'autre moitié est traitée à la mitomycine C ou aux rayons gamma. Des kératinocytes de poulet sont inoculés sur chaque sous clone traité à des densités de 10² à 10⁶ cellules par 1,5.10⁶ 3T3 par boite de 100mm de diamètre. Après 7-14 jours de culture, les cellules sont fixées et colorées à la rhodamine B. Le sous clone de 3T3, dénommé 3T3-J2, sur lequel les colonies de kératinocytes les plus nombreuses et de plus grande taille sont obtenues est considéré comme optimal ; il sera désormais utilisé pour cultiver les kératinocytes de poulet.

Kératinocytes épidermiques humains provenant de prépuce de nouveau-né (préparés suivant la méthode décrite dans Rheinwald et al., Cell, 6, 331-343, 1975).

### 1.2.2 Méthodes

### Culture cellulaire (méthode 1) :

La peau des pattes ou du corps est prélevée ; elle est lavée plusieurs fois dans du tampon phosphate salin (PBS), puis l'épiderme est séparé du derme par traitement enzymatique avec 0,5 mg/ml de thermolysine (Sigma) dans une solution tamponnée d'Hepes (HBS) soit pendant 45 minutes à 37°C ou toute la nuit à 4°C. L'épiderme est ensuite prélevé, émincé et désagrégé par incubation en présence de trypsine (0,25 %) et d'EDTA (0,02%) pendant deux fois 30 minutes à 37°C sous agitation dans un flacon de trypsination. La suspension cellulaire est centrifugée à 800 g pendant 5 minutes. Le culot cellulaire est repris dans du milieu de culture et les cellules sont inoculées dans des boîtes de Pétri recouvertes d'une monocouche de cellules murines 3T3 (2,3X10⁴ cellules/cm²) préalablement traitées par de la mitomycine C (4 µg/ml). Les boîtes sont ensuite placées dans un incubateur à 37°C sous 7,5% CO2 et 100% d'humidité. Le milieu de culture est constitué par un mélange 1/1 DMEM/F12 (Invitrogen) supplémenté par 10% de sérum de poulet (Invitrogen). Les additifs suivants sont également présents dans le milieu: 0,4 µg/ml hydrocortisone (Calbiochem), 5 µg/ml d'insuline bovine (Sigma), 2x10⁻⁹ M de 3,3',5-triiodo-L-thyronine (T3, Sigma), 10⁻¹⁰ M de toxine cholérique (ICN), 1,8x10⁻⁴ M d'adénine (Calbiochem). Le facteur de croissance épidermique (EGF) humain recombinant est ajouté à une concentration de 10 ng/ml au premier changement de milieu. Dans certains cas, des colonies de fibroblastes se développent malgré l'élimination du derme, mais elles peuvent être éliminées en traitant brièvement les cultures avec du PBS sans Ca++ et sans Mg++ ou avec de l'EDTA 0,02%; des cellules 3T3 de support nouvelles sont alors ajoutées et la culture continuée. Les cultures primaires sont transférées 7 à 19 jours après l'inoculation en fonction de la densité d'inoculation. On obtient alors des cultures secondaires. Les cellules sont congelées après le premier passage dans du milieu contenant 10 % de diméthyl sulfoxide (DMSO). La congélation est faite à raison de 1-3x10⁶ cellules/ml dans le milieu de culture décrit plus haut. Les cellules congelées peuvent être gardées indéfiniment dans l'azote liquide.

Les kératinocytes épidermiques humains sont mis en culture tel que décrit pour les cellules de poulet, sauf que le sérum de veau foetal est utilisé à la place du sérum de poulet.

### Culture cellulaire (méthode 2) : Culture de kératinocytes de poulet à l'interface liquide/air

Cette méthode est inspirée de l'article J.Y.Yi et al., Arch. Dermatol. Res., 2001, 293, 356-362. Elle a pour but d'obtenir une différentiation terminale plus complète. Les cellules 3T3 traitées ou non à la mitomycine sont mélangées à une matrice de collagène de type I (3.10⁵ cellules/ml) selon les instructions du fournisseur (Nitta gelatin, Tokyo), 200microlitres de cette mixture sont inoculés sur des filtres de polycarbonate (Millicell-pc Millipore, Bedford, MA). Les kératinocytes sont inoculés sur la matrice de collagène et cultivés immergés pendant 5-7jours dans le milieu de culture habituel (DMEM/F12 +sérum de poulet + additifs). Le filtre est ensuite placé à l'interface milieu/air et la culture est continuée pendant 14 jours.

### Microscopie en contraste de phase

On a utilisé un microscope de marque Nikon équipé d'une caméra numérique Nikon Coolpix.

### Coloration des gouttelettes lipidiques avec Oil Red O

Les cellules inoculées sur des lamelles couvre-objet en verre sont fixées dans un PBS froid contenant 3,7% de formaldéhyde pendant 15 minutes à température ambiante, puis incubées pendant une heure supplémentaire avec une solution de fixation nouvelle. Les lamelles couvre-objet en verre sont lavées à l'eau distillée et séchées à l'air. Les gouttelettes lipidiques sont colorées avec une solution contenant 0,3% de Oil Red O (Sigma) et 60% d'isopropanol pendant 1 heure. A la fin de l'incubation, les lamelles couvre-objet en verre sont lavées dans de l'eau distillée et incubées pendant 5 minutes dans du PBS contenant du réactif Hoechst 33258 (1µg/ml) et 0,1% du produit commercialisé par la société Sigma sous le nom commercial IGEPAL. Les lamelles couvre-objet en verre sont lavées une fois dans du PBS/IGEPAL, une fois dans de l'eau et montées sur lame dans du produit commercialisé par la société Calbiochem sous le nom commercial MOWIOL contenant 2,5% de DABCO (1,4-diazobicyclo-(2.2.2)-octane).

### 1.3 Résultats

Des cellules rondes attachées sur les cellules 3T3 sont visibles, après quelques heures d'inoculation, mais les colonies qui s'organisent clairement en un motif épithélial typique deviennent visibles après 5-6 jours d'inoculation, lorsque les cellules ont établi un contact avec la matière plastique. Les colonies déplacent la monocouche de cellules 3T3 autour d'elles comme le font les colonies formées par les kératinocytes humains. Ce qui apparaît comme le plus remarquable est que presque toutes les cellules contiennent une seule grosse gouttelette et quelques fois plusieurs petites, probablement composées de lipides (Figures 1A et B). De par leur origine épidermique, leur capacité à former un épithélium squameux stratifié, et étant donné que les kératinocytes aviaires sont connus pour contenir des gouttelettes lipidiques, on en conclut que les colonies sont composées de kératinocytes.

Les kératinocytes humains inoculés à une densité de 12,000 cellules/cm² dans un milieu supplémenté par 10% de sérum de poulet se multiplient aussi rapidement qu'ils le font dans le sérum de veau foetal. Ils ne développent jamais de gouttelettes lipidiques même après 12 jours en présence de 10% de sérum de poulet (Figures 1C et D). On en conclut que la formation de gouttelettes lipidiques est une propriété intrinsèque des kératinocytes de poulet et n'est pas induite dans des kératinocytes d'autres espèces par le sérum de poulet.

La coloration des cellules fixées par le produit Oil Red-O, qui est spécifique pour les lipides neutres montre que les gouttelettes réfringentes contiennent des lipides neutres (Figure 2A) ; une contre-coloration avec le réactif Hoechst 33258 met en évidence que la plupart des gouttelettes lipidiques des cellules sont périnucléaires (Figure 2B).

Lors de 3 expériences indépendantes, il a été mis en évidence qu'environ 500 colonies ont été formées à partir de 5x10⁵ cellules épidermiques désagrégées. Ceci correspond à une efficacité de formation de colonies de 10⁻³. L'efficacité d'ensemencement des kératinocytes de poulet prélevés directement d'épiderme est alors significativement inférieur à celui des kératinocytes humains, qui a été rapporté être de l'ordre de 1 à 10% mais est en général proche de 1%. Contrairement aux kératinocytes humains, qui atteignent des efficacités de formation de colonies très élevées lors des passages ultérieurs, l'efficacité d'ensemencement des kératinocytes de poulet ne dépasse jamais quelques pourcents.

Le temps de doublement des kératinocytes de poulet est mesuré dans la culture secondaire en comptant le nombre de cellules dans 15 colonies, toutes les 24 heures, à partir du 1^{er} jour jusqu'au 4^{ème} jour après inoculation. Les kératinocytes de poulet peuvent être facilement distinguées des cellules 3T3 avoisinantes étant donné qu'ils contiennent une gouttelette lipidique solitaire. Lorsque les colonies en plus forte croissance sont considérées, le temps de doublement de 16,8 heures est obtenu. Le temps de doublement décroît au cours des passages ultérieurs et les kératinocytes de poulet ne peuvent pas être mis en culture au delà de la 20^{ème} sous-culture.

La taille des kératinocytes de poulet est mesurée en introduisant une suspension de cellules dans un hématocytomètre, en photographiant les cellules et en mesurant leur diamètre. Les kératinocytes ont un diamètre typique de 10 µm, et sont donc plus petits que les kératinocytes humains, dont le diamètre le plus courant est de 12 µm. Cette différence peut s'expliquer par le fait que la taille du génome de poulet est seulement un tiers de celui de l'homme (1,25pg versus 3,5pg/génome haploïde). Les fibroblastes de poulet, avec un diamètre typique de 12 µm, sont aussi plus petits que les fibroblastes humains dont le diamètre le plus commun est de 15,6 µm.

### Exemple 2 : Rôle du sérum de poulet dans le processus de multiplication des kératinocytes de poulet

### 2.1 Introduction

Le sérum de veau foetal ne permet pas la multiplication des kératinocytes de poulet, soit parce qu'il contient un inhibiteur de multiplication, soit parce qu'il est dépourvu d'un facteur promouvant la croissance.

De façon à discriminer entre ces alternatives, les kératinocytes de poulet sont cultivés pendant 10 jours soit en présence de 10% de sérum de veau foetal supplémenté par des concentrations croissantes de sérum de poulet, soit en présence de 10% de sérum de poulet supplémenté par des concentrations croissantes de sérum de veau foetal. A la fin de l'expérience, les cellules sont fixées et colorées avec de la rhodamine B

### 2.2 Matériel et Méthodes

### 2.2.1 Matériel

Le matériel biologique utilisé est celui décrit dans l'Exemple 1.

### 2.2.2 Méthodes

Le protocole de culture cellulaire est utilisé selon celui décrit dans l'Exemple 1.

### Coloration à la rhodamine B

Les cultures sont lavées deux fois avec du PBS et fixées dans du PBS contenant 10% de glutaraldéhyde pendant 15 minutes à température ambiante. Les cellules sont alors colorées grâce à 1% de rhodamine B dans de l'eau pendant 15 minutes à température ambiante sous agitation douce. Les cultures sont lavées dans de l'eau et sont ensuite séchées à l'air.

### 2.3 Résultats

Dans la Figure 3, il apparaît clairement que le sérum de poulet permet la multiplication des kératinocytes et que cette faculté est fonction de la dose de sérum de poulet, lorsqu'il est ajouté à 10% de sérum de veau foetal. L'activité promotrice de croissance de 10% de sérum de poulet n'est pas affectée par l'addition de quantités croissantes de sérum de veau foetal. On en conclut que le sérum de poulet contient un facteur nécessaire pour la multiplication des kératinocytes de poulet ; ce facteur étant absent du sérum de veau foetal.

Etant donné que les kératinocytes de poulet contiennent des lipides, on pouvait supposer que l'activité promotrice de croissance du sérum de poulet est liée à l'absorption des lipides dans le sérum. L'expérience illustrée par la figure 4A montre que le sérum de poulet dépourvu de lipides par l'action de solvants retient sa faculté de promouvoir la multiplication des kératinocytes de poulet. Par conséquent, l'activité promotrice de croissance n'est pas due à un lipide. Les cellules qui se multiplient, en particulier autour des colonies, retiennent les lipides lorsqu'elles croissent dans du sérum délipidé et sont donc capables de synthétiser des lipides (Figure 4B).

### Exemple 3: Les kératinocytes de poulet en culture synthétisent des alpha-kératines

### 3.1 Introduction

Une des propriétés des cellules épidermiques est leur capacité à synthétiser des kératines en abondance. Les kératinocytes de poulet sont mis en culture sur des lamelles couvre-objet en verre pendant 2 jours et sont fixés. Les cellules sont alors incubées en présence d'un anticorps anti-α-pankératine et examinées par microscopie par fluorescence indirecte.

On a analysé les kératines des cellules épidermiques de poulet en culture par immunodétection de type Westem-blot. Les kératines sont extraites à partir de cultures confluentes ; séparées par électrophorèse sur gel en une dimension ; transférées sur nitrocellulose et colorées à l'aide d'un anticorps anti-α-pankératine.

### 3.1 Matériel et Méthodes

### 3.1.1 Matériel

Le matériel biologique est celui décrit dans l'Exemple 1.

### 3.2.1 Méthodes

### Coloration par immunofluorescence indirecte

Les cellules sont inoculées sur des lamelles couvre-objet en verre. Le lendemain, les cellules sont lavées 2 fois dans du PBS, puis fixées dans un mélange 1 :1 d'acétone : méthanol pendant 20 minutes à -20°C et séchées à l'air. Les lamelles couvre-objet en verre sont incubées dans du PBS contenant 5% d'albumine sérique bovine (BSA) pendant 10 minutes à température ambiante pour bloquer les sites non-spécifiques.

Pour la coloration de l'α-kératine, les cellules sont incubées pendant 1 heure à température ambiante dans 5% de BSA/PBS contenant un anticorps anti-pankératine monoclonal de souris (dilution de 1 :100, MS-744-A0, NeoMarkers), avant d'être lavées 3 fois pendant 5 minutes à chaque fois avec du PBS contenant 0,1% du détergent non-ionique IGEPAL CA 630 (Sigma). Les cellules sont alors incubées pendant 1 heure à température ambiante en présence de 5% de BSA/PBS contenant des IgG anti-souris de chèvre liées à la biotine (dilution de 1 : 1 000, Jackson ImmunoResearch). Les lamelles couvre-objet en verre sont lavées et incubées pendant 1 heure avec de la streptavidine liée à de la cyanine-3 (dilution de 1 : 1000). A la fin de l'incubation, les lamelles couvre-objet en verre sont lavées pendant 5 minutes dans du PBS/IGEPAL avant d'être incubées pendant 5 minutes dans du PBS contenant du réactif Hoechst 33258 (1µg/ml) et 0,1% de IGEPAL. Les lamelles couvre-objet en verre sont lavées une fois dans du PBS/IGEPAL, une fois dans de l'eau et montées sur lame dans du mowiol contenant 2,5% de 1-4,diazobicyclo-(2.2.2)-octane (DABCO).

### Extraction des α-kératines et analyse par immunodétection (Western blotting)

Les cultures confluentes sont lavées 2 fois avec du PBS et broyées à l'aide d'un homogénéisateur de type Dounce. Les cellules sont homogénéisées après 20 rotations dans un tampon contenant 10 mM de Tris-HCl (pH 7,6), 1mM EDTA et un mélange d'inhibiteurs de protéase (Roche). L'homogénat est centrifugé à 8,000 g pendant 5 minutes à 4°C et le culot est lavé 2 fois avec du tampon Tris-EDTA et 2 fois avec du tampon Tris-EDTA contenant 2% de Nonidet P40. Les kératines dans le dernier culot sont mises en suspension dans une solution contenant 2% de SDS et 10mM de dithiothréitol à 37°c pendant 15 minutes avec l'aide d'un appareil à ultrasons (2x15 secondes). Les échantillons sont alors chauffés à 100°C pendant 2 minutes et centrifugés pendant 5 minutes à 8,000 g pour éliminer les résidus insolubles.

Les α-kératines sont séparées par électrophorèse sur un gel de 8% polyacrylamide dénaturant (proportion en poids acrylamide : bisacrylamide de 29 :1) et électroéluées sur nitrocellulose. La membrane est traitée toute la nuit à 4°C dans du PBS contenant 5% de lait lyophilisé écrémé et 0,1% de Tween 20 (PBST). Les alpha-kératines sont colorées avec un anticorps anti-pankératine consistant en un mélange de 2 anticorps monoclonaux, l'un étant dirigé contre les kératines de type I, et l'autre étant dirigé contre les kératines de type II (PRO61835, Research Diagnostics Inc). L'anticorps est utilisé à une dilution de 1 :500, et la membrane est lavée par du PBST avant d'être incubée avec des IgG diluées 1 :2000 anti-souris d'âne conjuguées à la peroxydase de raifort (Amersham). Les incubations d'anticorps durent 1 heure à température ambiante. Les bandes correspondant à la kératine sont visualisées par chimioluminescence (ECL+kit, Amersham).

### 3.3 Résultats

Tous les kératinocytes de poulet en culture sont fortement marqués, mais les cellules 3T3 ne sont pas marquées (Figure 5A). Aucun marquage n'est observé lorsque l'anticorps spécifique est omis.

Les résultats issus d'immunodétection sont illustrés dans la figure 5B. Les protéines colorées par l'anticorps sont séparées en trois doublets majeurs (a-c), dont les poids moléculaires respectifs sont d'environ de 50, 57 et 63 kD. Les doublets a et c sont également présents dans la peau de poulet, mais le doublet b ne l'est pas. Le profil général des kératines extraites à partir d'intestin de poulet, un épithélium non-kératinisant, ne coïncide pas avec les cellules épidermiques, ni avec l'épiderme. Etant donné que les cellules en culture contiennent deux doublet de kératines dont les poids moléculaires coïncident avec ceux des kératines épidermiques, la Demanderesse en conclut que les cellules en culture synthétisent des kératines épidermiques. Les deux kératines de poids moléculaire élevé (doublet c dans Figure 5B) sont particulièrement pertinentes étant donné que les kératines à poids moléculaire élevé sont considérées comme spécifiques des couches externes des épithélium squameux stratifiés. Quatre α-kératines sont davantage caractérisées (voir la suite)

### Exemple 4 : Les kératinocytes de poulet forment des enveloppes cornifiées en culture

### 4.1 Introduction

Une des propriétés distinctives des kératinocytes épidermiques de mammifère est leur capacité à suivre le processus de différentiation terminale, dans lequel leur noyau est détruit et pendant lequel ils développent une enveloppe cellulaire cornifiée qui est insoluble en présence de dodécyl sulfate de sodium et de 2-mercaptoéthanol.

### 4.2 Matériel et méthodes

Le matériel biologique et les méthodes utilisés dans cet exemple sont ceux décrits dans l'Exemple 1.

### 4.3 Résultats

Lorsque l'on observe le centre des colonies de kératinocytes de poulet dont la taille excède plusieurs centaines de cellules on constate qu'ils contiennent presque toujours des cellules de grande taille qui recouvrent chacune plusieurs cellules basales (Figure 6A et B). Ces squames sont rapidement libérés dans le milieu, elles contiennent toujours des gouttelettes réfringentes, probablement composées de lipides. Les squames de poulet sont plus minces et sont environ deux fois plus grosses que les squames humaines (plus grande longueur de 100 µm *versus* 50 µm) (Figure 6B). Lorsque les colonies épidermiques de poulet sont prélevées et placées dans une solution contenant 2% de SDS et 2% de 2-mercaptoéthanol et chauffées jusqu'à 100°C, la plupart des cellules se dissolvent mais les enveloppes cornifiées restent insolubles et gardent encore des gouttelettes lipidiques (Figure 6C). Une grande partie des enveloppes préparées directement à partir d'épiderme de poulet gardent également des lipides après ébullition dans du SDS/2-mercaptoéthanol. Ces lipides sont extractibles avec du méthanol/choroforme.

### Exemple 5 : Les kératinocytes de poulet contiennent la p63

La protéine p63, qui fait partie de la famille du gène codant pour la protéine p53, se trouve en abondance dans la couche basale de nombreux épithéliums. Dans l'épiderme humain, p63 se trouve uniquement dans des cellules ayant un potentiel de prolifération élevé. L'invalidation du gène codant pour p63 aboutit à une absence de tout épithélium squameux stratifié et la p63 est considérée comme un marqueur des cellules souches de kératinocytes.

De façon à déterminer si les kératinocytes de poulet contiennent la p63, les cellules en croissance rapide sont ensemencées sur des lamelles en verre et observées le lendemain par microscopie par immunofluorescence, en utilisant l'anticorps anti-p63 monoclonal 4A4.

### 5.1 Matériel et Méthodes

### 5.1.1 Matériel

Le matériel biologique est décrit dans l'Exemple 1.

### 5.1.2 Méthodes

La culture cellulaire et la technique de coloration par immunofluorescence indirecte sont celles décrites respectivement dans les Exemple 1 et 3.

### Marquage de la p63

Les cellules sont incubées toute la nuit à 4°C en présence d'un anticorps monoclonal anti- p63 humain 4A4, dilué à 1 : 500. Le reste du protocole est identique à celui utilisé pour la coloration de la kératine sauf que l'anticorps secondaire est utilisé à une dilution de 1 : 200. Les cellules sont photographiées en utilisant un microscope à fluorescence Nikon E600 équipé d'un appareil photographique digital Nikon.

### 5.2 Résultats

Toutes les cellules examinées présentent une forte coloration nucléaire pour p63. Au contraire, les cellules 3T3 ne présentent soit aucun marquage ou parfois un faible marquage cytoplasmique (Figure 7).

### Exemple 6 : Caractérisation des kératinocytes de poulet par microscopie életronique

### 6.1 Matériel et Méthodes

On prépare des cultures monocouches de kératinocytes de poulet. Lorsque les cellules sont à 2/3 confluentes, les cellules 3T3 sont enlevées à l'aide d'EDTA, et les kératinocytes sont transférés dans des petites boites de Pétri en absence de cellules de support 3T3. Le lendemain, les cellules sont fixées et traitées pour être observées par microscopie électronique.

### Microscopie électronique

Les cellules sont lavées dans 0.1 M de cacodylate à pH 7,3, fixées pendant 90 minutes avec 5% de paraformaldéhyde et 5% de glutaraldéhyde dans 0.1 M de cacodylate de sodium à pH 7,2 et observées par microscopie électronique classique. Des sections ultra-minces, colorées avec de l'acétate d'uranyle et du citrate de plomb, sont examinées à 80 kV à l'aide d'un microscope électronique Jeol JEM-1010 (JEOL Ltd, Tokyo, Japon).

### 6.2 Résultats

La figure 8 montre un ensemble de micrographies de cellules contenant des gouttelettes lipidiques. Deux micrographies (A et B) montrent une cellule typique avec une gouttelette lipidique périnucléaire unique qui est enchâssée dans le noyau. Ainsi, la plupart des noyaux présentent une forme en croissant. Les deux autres photographies montrent une cellule avec de multiple petites gouttelettes lipidiques cytoplasmiques (C) et une autre cellule avec des gouttelettes lipidiques à l'intérieur du noyau (D) ; ces deux caractéristiques étant communément présentes. La figure 9 montre des caractéristiques cellulaires épidermiques typiques. La photographie (A) met en évidence la présence de desmosomes. La photographie (B) montre une cellule basale et suprabasale ayant leur cytoplasme rempli par des tonofilaments (kératine).

### Exemple 7 : Identification de produits de kératinocyte spécifiques par séquençage aléatoire

### 7.1 Introduction

Une banque d'ADNc est construite à partir de cultures de kératinocyte de poulet confluentes. La séquence de nucléotidique de l'extrémité 5' issue de 163 clones aléatoires est déterminée avec l'aide d'un séquenceur automatique. Les banques de données GenBank et de protéines sont alors utilisées pour des recherches d'homologie avec les séquences d'ADNc.

### 7.2 Matériel et Méthodes

### Construction de la banque d'ADNc et séquençage de nucléotides

L'ARN total est purifié à partir de kératinocytes de poulet en culture en utilisant le réactif Tripure Isolation Reagent (Roche). Les ARN poly(A)⁺ sont isolés sur cellulose oligo(dT) en utilisant le kit de purification pour ARNm Micropoly(A) Purist^{™}(Ambion). Une banque d'ADNc est préparée à partir de 1,7µg de ARN poly(A)⁺ en utilisant le SuperScript^{™} Plasmid System (Life Technologies). La synthèse du premier brin est initiée à l'aide d'une amorce contenant une séquence oligo(dT) suivie par un site *Not*I*.* Un adaptateur *Sal*I est ajouté à chaque extrémité de l'ADNc après la synthèse du 2^{nd} brin. La digestion avec *Not*I génère un bout cohésif à l'extrémité 3', tandis que le bout cohésif *Sal*I reste à l'extrémité 5'. Les ADNc sont liés au plasmide pSport1 préalablement coupé avec *Sal*I et *Not*I, et introduit par électroporation dans des bactéries *E.Coli* DH10B (Life Technologies) électrocompétentes.

Le séquençage aléatoire de l'extrémité 5' des clones d'ADNc est réalisé en utilisant une amorce qui s'hybride au plasmide, en amont du site *Sal*I. Pour étendre la séquence, les amorces internes spécifiques sont utilisées : pour l'α-kératine 5/6 :
SEQ ID NO : 1 5'-AGTGGATTCTATGGACCTGC-3',
SEQ ID NO : 2 5'-AACTGAGCAGCTTGCTGGC-3',
SEQ ID NO : 3 5'-CAGTCCAGGTACGAAGAGC-3'
SEQ ID NO : 4 5'-TAGGATCGGGATATGGAAGC-3' ;
pour l'α-kératine 15 :
SEQ ID NO : 5 5'-GGCTTTGATGCTATCTGTGC-3',
SEQ ID NO : 6 5'-GTGGAGTCTGACATCAACG-3'
SEQ ID NO : 7 5'-AGAACTGAGACGCACGATGC-3' ;
pour la β-kératine K :
SEQ ID NO : 8 5'-ACTAGGATGTTACTGCGTGG-3'.

Le séquençage est réalisé sur un appareil PCR GeneAmp 2400 (Perkin-Elmer) en présence de didéoxynucléotides fluorescents. Les conditions de PCR sont 30 cycles à 96°C pendant 30 secondes, 50°C pendant 15 secondes et 60°C pendant 4 minutes. L'électrophorèse et la détection des pics de fluorescence sont mises en oeuvre sur un séquenceur automatique (ABI PRISM 310 Genetic Analyzer). La séquence est déterminée en utilisant le logiciel SeqEd v1.0.3.

### 7.3 Résultats

50 clones ne présentent aucune homologie avec les séquences des bases de données. 15 clones présentent des homologies avec des protéines hypothétiques. 86 clones codent pour des protéines universelles ayant des fonctions connues. 12 clones codent pour des protéines connues pour participer à des fonctions spécifiques des kératinocytes. La Demanderesse en conclut que dans les kératinocytes de poulet, environ 40% (65/163) des ARNm codent pour des protéines de fonction inconnue et qu'environ 12% (12/98) des ARNm sont spécifiques de ce type de cellules. Au total 7 clones spécifiques de kératinocytes codent pour des kératines (Tableau III). Les clones 6-8 codent pour la même alpha-kératine, alors que les clones 9-11 codent pour différentes α-kératines et le clone 12 code pour une β-kératine. La séquence entière de nucléotides est déterminée pour les clones 6 et 9-12.

### Exemple 8 : Infection des kératinocytes de poulet en culture par le virus de la maladie de Marek (MDV)

### 8.1 Matériel et Méthodes

Les cellules infectées, issues de cellules primaires de peau de poulets (essentiellement fibroblastes) ou lymphocytes sanguins infectés par MDV-1, sont ensemencées sur des kératinocytes de poulet en culture subconfluente et incubées pendant 1 heure à 37°C. Après l'adsorption, l'inoculum est prélevé par aspiration et les mono-couches de kératinocytes de poulet sont lavées 3 fois avec un tampon phosphate salin (PBS). Le milieu de culture cellulaire est ajouté et les cellules sont incubées pendant une semaine à 37°C.

Les virions MDV complètement enveloppés sont libérés dans le milieu de culture cellulaire qui peut être récupéré et lyophilisé. Pour la lyophilisation, le milieu de culture cellulaire contenant les virions MDV est congelé à -60°C, séché sous vide à 38°C pendant 24 heures, séché pendant 15 heures à 21°C, et stocké à 4°C sous vide.

Les cultures de kératinocytes de poulet infectés sont prélevées dans du PBS, ou dans du PBS contenant 0,2% d'EDTA, ou dans une solution de stabilisation « SPGA » consistant en 2,18x10⁻¹M de saccharose, 7,2x10⁻³M de phosphate de dipotassium, 3,8x10⁻³M de phosphate de potassium, 4,9x10⁻³M de glutamate de sodium, 1% de poudre d'albumine bovine, ou dans du SPGA contenant 0,2% d'EDTA (SPGA-EDTA), ou dans du SPGA-EDTA avec 10% de sorbitol, ou dans un milieu de culture cellulaire avec 10% de sorbitol. Les suspensions cellulaires sont alors rompues par ultrasons pendant 2 minutes et centrifugées à 2000g pendant 20 minutes et les surnageants sont récupérés en tant que préparations virales dépourvues de cellules. Les virus sont extraits des kératinocytes infectés par 3 cycles de congélation/décongélation, et les fluides de surnageant sont récupérés. Les virus dépourvus de cellules peuvent être lyophilisés comme décrit ci-dessus.

## Revendications

1. Culture de kératinocytes aviaires **caractérisée en ce qu'**elle comporte un milieu de culture qui comprend du sérum de poulet et un support de culture comprenant des fibroblastes inactivés.

2. Culture de kératinocytes selon la revendication 1 **caractérisée en ce qu'**elle comporte un milieu de culture qui comprend du sérum de poulet et un support de culture comprenant des cellules 3T3 de la lignée cellulaire enregistrée dans la collection ATCC sous le numéro CCL92 inactivées.

3. Culture de kératinocytes selon la revendication 2, **caractérisée en ce que** la proportion en poids de sérum de poulet par rapport au poids total du milieu de culture est comprise entre 0,5 et 50%.

4. Culture de kératinocytes selon la revendication 2, **caractérisée en ce que** les cellules 3T3 sont des cellules issues des cellules 3T3 de la lignée cellulaire enregistrée dans la collection ATCC sous le numéro CCL92 par un procédé comportant les étapes d'inoculation dans des puits, de culture, transfert, culture, détachement par trypsination, congélation de la moitié de chaque sous clone, traitement de l'autre moitié à la mitomycine C ou aux rayons gamma incubation de kératinocytes de poulet sur chaque sous clone traité, culture, fixation et coloration à la rhodamine B, sélection du sous clone sur lequel les colonies de kératinocytes les plus nombreuses et de plus grande taille sont obtenues.

5. Culture de kératinocytes selon la revendication 2, **caractérisée en ce que** les kératinocytes sont des kératinocytes de poulet.

6. Procédé de culture de cellules épidermiques ou kératinocytes aviaires, ledit procédé comprenant les étapes consistant à :
(i) préparer une suspension de kératinocytes ;
(ii) inoculer lesdites cellules dans un milieu de culture comprenant du sérum de poulet et un support de culture comprenant des fibroblastes inactivés ;
(iii) incuber lesdites cellules dans ce milieu de culture.

7. Procédé selon la revendication 6, **caractérisé en ce que** le milieu de culture est tel que défini à l'une des revendications 2 à 4.

8. Procédé de culture selon la revendication 6 ou la revendication 7, **caractérisé en ce qu'**à l'issue de l'étape (iii) les cellules issues de la culture sont récupérées et on leur applique un nouveau cycle de culture suivant les étapes (ii) et (iii) du procédé selon la revendication 6.

9. Procédé de culture selon la revendication 8, **caractérisé en ce qu'**il comporte au plus 30 cycles de culture cellulaire.

10. Procédé de propagation d'un virus *in vitro,* ledit procédé comprenant les étapes consistant à :
(i) infecter des kératinocytes aviaires avec la souche du virus ;
(ii) mettre en culture les kératinocytes infectés suivant le procédé des revendications 6 à 9 ;
(iii) extraire les virions produits à l'étape (ii).

11. Procédé selon la revendication 10, **caractérisé en ce qu'**il comprend en outre une étape de lyophilisation des virions.

12. Procédé selon la revendication 10 ou la revendication 11, **caractérisé en ce que** les kératinocytes sont des cellules d'épiderme de poulet.

13. Procédé selon l'une quelconque des revendications 10 à 12 **caractérisé en ce que** le virus est choisi parmi le MDV et le FPV.

14. Procédé de préparation d'un médicament destiné à la prévention ou au traitement de la maladie de Marek, **caractérisé en ce qu'**il comporte un procédé selon l'une des revendications 10 à 13.

## Claims

1. Culture of avian keratinocytes, **characterised in that** it comprises a culture medium which contains chicken serum and a culture substrate comprising inactivated fibroblasts.

2. Culture of keratinocytes according to claim 1, **characterised in that** it comprises a culture medium which contains chicken serum and a culture substrate comprising inactivated 3T3 cells of the cell line registered in the ATCC collection under the number CCL92.

3. Culture of keratinocytes according to claim 2, **characterised in that** the proportion by weight of chicken serum based on the total weight of the culture medium is between 0.5 and 50%.

4. Culture of keratinocytes according to claim 2, **characterised in that** the 3T3 cells are cells obtained from the 3T3 cells of the cell line registered in the ATCC collection under the number CCL92 by a process comprising the steps of inoculation into wells, culturing, transfer, culturing, detachment by trypsination, freezing half of each sub-clone, treating the other half with mitomycin C or with gamma rays: incubating chicken keratinocytes on each treated sub-clone, culturing, fixing and staining with rhodamine B, and selecting the subclone on which the largest and most numerous colonies of keratinocytes are obtained.

5. Culture of keratinocytes according to claim 2, **characterised in that** the keratinocytes are chicken keratinocytes.

6. Process for culturing avian epidermal cells or keratinocytes, the said process comprising the steps of:
(i) preparing a suspension of keratinocytes;
(ii) inoculating the cells into a culture medium comprising chicken serum and a culture substrate comprising inactivated fibroblasts;
(iii) incubating the said cells in this culture medium.

7. Process according to claim 6, **characterised in that** the culture medium is as defined in one of claims 2 to 4.

8. Culturing process according to claim 6 or claim 7, **characterised in that** at the end of step (iii) the cells obtained from the culture are recovered and subjected to a fresh culture cycle according to steps (ii) and (iii) of the process according to claim 6.

9. Culturing process according to claim 8, **characterised in that** it comprises not more than 30 cell culture cycles.

10. Process for propagating a virus *in vitro,* the said process comprising the steps of:
(i) infecting avian keratinocytes with the strain of virus;
(ii) culturing the infected keratinocytes using the process according to claims 6 to 9;
(iii) extracting the virions produced in step (ii).

11. Process according to claim 10, **characterised in that** it further comprises a step of lyophilising the virions.

12. Process according to claim 10 or claim 11, **characterised in that** the keratinocytes are chicken epidermis cells.

13. Process according to any one of claims 10 to 12, **characterised in that** the virus is selected from among MDV and FPV.

14. Process for preparing a medicament intended for preventing or treating Marek's disease, **characterised in that** it comprises a process according to one of claims 10 to 13.

## Patentansprüche

1. Aviäre Keratinozytenkultur, **dadurch gekennzeichnet, dass** sie ein Kulturmedium umfasst, das Hühnerserum und einen Träger für die Kultur enthaltend inaktivierte Fibroblasten enthält.

2. Keratinozytenkultur gemäß Anspruch 1, **dadurch gekennzeichnet, dass** sie ein Kulturmedium umfasst, das Hühnerserum und einen Träger für die Kultur enthaltend inaktivierte 3T3-Zellen der Zelllinie, die in der ATCC-Sammlung unter der Nummer CCL92 registriert ist, enthält.

3. Keratinozytenkultur gemäß Anspruch 2, **dadurch gekennzeichnet, dass** der Gewichtsanteil des Hühnerserums in Bezug auf das Gesamtgewicht des Kulturmediums zwischen 0,5 und 50% liegt.

4. Keratinozytenkultur gemäß Anspruch 2, **dadurch gekennzeichnet, dass** die 3T3-Zellen Zellen sind, die von 3T3-Zellen der Zelllinie, die in der ATCC-Sammlung unter der Nummer CCL92 registriert ist, durch ein Verfahren erhalten werden, enthaltend die Schritte: Inokulation in Vertiefungen, Kultur, Transfer, Kultur, Ablösen durch Trypsinieren, Einfrieren eines Teils eines jeden Subklons, Behandlung des anderen Teils mit Mitomycin C oder Gammastrahlen, Inkubation von Hühnerkeratinozyten auf jedem der behandelten Subklone, Kultur, Fixierung und Anfärbung mit Rhodamin B, Auswahl des Subklons, auf dem die Keratinozytenkolonien am zahlreichsten und mit größtem Wuchs erhalten werden.

5. Keratinozytenkultur gemäß Anspruch 2, **dadurch gekennzeichnet, dass** die Keratinozyten Hühnerkeratinozyten sind.

6. Verfahren der Kultivierung von epidermalen Zellen oder aviären Keratinozyten, wobei das Verfahren die Schritte umfasst:
(i) Herstellen einer Keratinozytensuspension;
(ii) Inokulieren dieser Zellen in einem Kulturmedium, das Hühnerserum und einen Träger für die Kultur enthaltend inaktivierte Fibroblasten enthält;
(iii) Inkubieren dieser Zellen in diesem Kulturmedium.

7. Verfahren gemäß Anspruch 6, **dadurch gekennzeichnet, dass** das Kulturmedium jenes ist wie in einem der Ansprüche 2 bis 4 definiert.

8. Verfahren der Kultivierung gemäß Anspruch 6 oder Anspruch 7, **dadurch gekennzeichnet, dass** am Ende des Schrittes (iii) die aus der Kultur stammenden Zellen gewonnen werden und man diese einem neuen Kultivierungszyklus nach den Schritten (ii) und (iii) gemäß Anspruch 6 unterwirft.

9. Verfahren der Kultivierung gemäß Anspruch 8, **dadurch gekennzeichnet, dass** es höchstens 30 Zyklen der Zellkultivierung enthält.

10. Verfahren zur Vermehrung eines Virus *in vitro,* wobei das Verfahren die Schritte enthält:
(i) Infizieren von aviären Keratinozyten mit dem Virusstamm;
(ii) Kultivierung der infizierten Keratinozyten nach dem Verfahren der Ansprüche 6 bis 9;
(iii) Gewinnen der in Schritt (ii) hergestellten Virionen.

11. Verfahren nach Anspruch 10, **dadurch gekennzeichnet, dass** es darüber hinaus einen Schritt der Lyophilisierung von Virionen umfasst.

12. Verfahren nach Anspruch 10 oder Anspruch 11, **dadurch gekennzeichnet, dass** die Keratinozyten Zellen der Epidermis von Hühnern sind.

13. Verfahren nach irgend einem der Ansprüche 10 bis 12, **dadurch gekennzeichnet, dass** das Virus ausgewählt ist aus MDV (Marek's disease virus) und FPV (Fowlpox Virus).

14. Verfahren zur Herstellung eines Arzneimittels zur Vorbeugung oder Behandlung der Marek'schen-Krankheit, **dadurch gekennzeichnet, dass** es ein Verfahren gemäß einem der Anspruche 10 bis 13 umfasst.
